# EUROPÄISCHE PATENTANMELDUNG

(11) **EP 1 512 386 A1**
(43) Veröffentlichungstag der Anmeldung: **09.03.2005**
(21) Anmeldenummer: 04405501.0
(22) Anmeldetag: 06.08.2004
(51) Int. Cl.: A61F 9/06

(54) **Schweisserschutzmaske mit Beleuchtungseinrichtung**

(30) Priorität: 02.09.2003 CH 15012003
(71) Anmelder: OPTREL AG, 9630 Wattwil (CH)
(72) Erfinder: Steinemann, Lukas, 8645 Jona (CH)
(74) Vertreter: Frei Patent Attorneys

(57) **Zusammenfassung**

Eine Schweisserschutzmaske (1) weist eine Beleuchtungseinrichtung (3) zur Ausleuchtung eines Arbeitsbereiches auf. Die Beleuchtungseinrichtung (3) weist Leuchtmittel (5), Erfassungsmittel (8) zur Erfassung einer Umgebungslichtintensität, einen Energiespeicher (7) zur elektrischen Speisung der Beleuchtungseinrichtung (3) und Steuermittel (6) zur Steuerung einer Intensität eines vom Leuchtmittel (5) abgestrahlten Lichtes nach Massgabe der Umgebungslichtintensität auf.

In einer bevorzugten Ausführungsform der Erfindung sind die Steuermittel (6) eingerichtet, um mittels einer Flackerschaltung einen Schweissvorgang zu detektieren und das Leuchtmittel (5) während des Schweissvorgangs auszuschalten.

## Beschreibung

Die Erfindung bezieht sich auf eine Schweisserschutzmaske mit Beleuchtungseinrichtung gemäss dem Oberbegriff des Patentanspruches 1, sowie auf eine Beleuchtungseinrichtung und einen Adapter zur Halterung der Beleuchtungseinrichtung gemäss den Patentansprüchen 9 und 10.

### STAND DER TECHNIK

Es ist beispielsweise im Automobil- oder Schiffsbau üblich, Lampen zur Beleuchtung eines Arbeitsbereiches eines Schweissers anzuordnen. Dies ist mit einem grossen Installations- und Verkabelungsaufwand verbunden. Eine Schweisserschutzmaske mit einer eigenen Beleuchtungseinrichtung ist beispielsweise aus DE 31 41 228 A1 bekannt. Eine Halogenlampe ist an der Schweisserschutzmaske angebracht und wird durch einen Transformator oder ein Schweissgerät über ein Kabel und einen Fuss- oder Handschalter gespeist. Auch bei dieser Art der Beleuchtung ist eine Verkabelung zur Speisung der Lampe notwendig, welche den Einsatz der Lampe verumständlicht.

### DARSTELLUNG DER ERFINDUNG

Es ist deshalb Aufgabe der Erfindung, eine Schweisserschutzmaske der eingangs genannten Art zu schaffen, welche die Nachteile des Stands der Technik behebt. Es ist ferner Aufgabe der Erfindung, eine entsprechende Beleuchtungseinrichtung und einen Adapter zur Halterung der Beleuchtungseinrichtung zu schaffen.

Diese Aufgaben werden gelöst durch die Gegenstände der entsprechenden unabhängigen Patentansprüche.

Die erfindungsgemässe Schweisserschutzmaske weist also eine Beleuchtungseinrichtung mit einem Leuchtmittel, einem Erfassungsmittel zur Erfassung einer Umgebungslichtintensität, einem Energiespeicher zur elektrischen Speisung der Beleuchtungseinrichtung, und einem Steuermittel zur Steuerung einer Intensität eines vom Leuchtmittel abgestrahlten Lichtes z.B. nach Massgabe der Umgebungslichtintensität auf.

Dadurch wird es aufgrund des Energiespeichers möglich, die Beleuchtungseinrichtung ohne Verkabelung zu einer von der Maske abgesetzten elektrischen Speisung zu betreiben, wobei durch die Steuerung der Lichtintensität ein Energieverbrauch der Beleuchtungseinrichtung, verglichen mit einem Dauerbetrieb mit konstanter Lichtintensität, verkleinert wird.

In einer bevorzugten Ausführungsform des Erfindungsgegenstandes sind die Steuermittel eingerichtet, bei zunehmender Umgebungslichtintensität die Intensität des abgestrahlten Lichtes zu verringern. Dies kann auf verschiedene Arten geschehen. Die Intensität des abgestrahlten Lichtes wird dabei jeweils bei zunehmender Intensität des Umgebungslichts monoton sinkend und/oder schrittweise auf einen Wert Null verringert.

In einer bevorzugten Ausführungsform der Erfindung wird das Leuchtmittel ausgeschaltet, falls die gemessene Intensität des Umgebungslichts einen vorgegebenen Wert überschreitet, oder falls eine Flackerschaltung einen Schweissvorgang detektiert. Dadurch ist einerseits gewährleistet, dass eine ausreichende Beleuchtung des Arbeitsbereichs vorliegt, und andererseits der Energiespeicher nicht ohne Notwendigkeit belastet wird.

Das Leuchtmittel weist vorzugsweise eine oder mehrere Leuchtdioden, insbesondere weisse respektive blaue Leuchtdioden auf. Dadurch wird der Energieverbrauch der Beleuchtungseinrichtung weiter verringert. Die Leuchtdioden werden zur weiteren Energieeinsparung vorzugsweise gepulst betrieben, was innerhalb gewisser Grenzen ohne Verringerung ihrer wahrgenommenen Leuchtintensität möglich ist. Falls die Intensität des von den Leuchtdioden abgestrahlten Lichts geregelt werden soll, geschieht dies vorzugsweise durch Veränderung der Spannung, mit welcher sie angesteuert werden. Bei einer gepulsten Ansteuerung wird vorzugsweise eine Modulation der Pulsung durch Pulsbreiten- oder Pulsfrequenzmodulation vorgenommen..

In einer weiteren bevorzugten Ausführungsform des Erfindungsgegenstandes ist die Beleuchtungseinrichtung als herausnehmbare Einheit, insbesondere als mechanisch selbständige Baueinheit und als elektrisch autonome Einheit ausgestaltet. Diese Einheit umfasst also den Energiespeicher, die Erfassungsmittel, die Steuermittel und die Leuchtmittel. Die Einheit kann zum Betrieb in eine Schweisserschutzmaske eingesetzt werden.

Die als selbständige Einheit ausgestaltete Beleuchtungseinrichtung ist vorzugsweise in einen Adapter zur Halterung einer Blendschutzvorrichtung in der Schweisserschutzmaske einsetzbar. Dieses Einsetzen geschieht beispielsweise durch eine einrastendende und wieder lösbare Schnapp- oder Clipverbindung, oder mit Hilfe von verschiebbaren Feststellelementen, die von Hand eingerastet und wieder geöffnet werden können. Eine Blendschutzvorrichtung ist ein Schwarzglas oder eine elektro-optische Einheit. Die elektrooptische Einheit mit einer im Grundzustand durchsichtigen Sichtscheibe zeichnet sich dadurch aus, dass beim Detektieren eines Lichtbogens die Sichtscheibe automatisch verdunkelt und der Schweisser vor Blendung geschützt wird. Moderne Blendschutzvorrichtungen weisen als Energiespeicher Akkus oder Batterien auf, die mit Solarzellen gespeist und dadurch nicht mit anderen Mitteln aufgeladen werden müssen. Die Detektion eines Lichtbogens geschieht in bekannter Weise mit einer sogenannten Flackerschaltung, wie sie auch in der vorliegenden Erfindung eingesetzt werden kann. Eine solche Flackerschaltung detektiert das Auftreten eines Schweissvorgangs aufgrund charakteristischer Eigenschaften der dabei auftretenden und gemessenen Lichtintensität, beispielsweise aufgrund eines hohen Signalanteils in Frequenzbereichen über 200 Hz. Um Blendschutzvorrichtungen und Schweisserschutzmasken verschiedener Bauart miteinander kombinieren zu können, werden mechanische Adapter eingesetzt, die eine lichtdichte sowie form- und kraftschlüssige mechanische Verbindung zwischen einer Blendschutzvorrichtung und einer Schweisserschutzmaske herstellen. Die Anordnung der Beleuchtungseinrichtung in einem solchen Adapter erlaubt somit auch eine flexible Verwendung in verschiedenen Schutzmasken und eine einfache Austauschbarkeit der Beleuchtungseinrichtung.

Die Beleuchtungseinrichtung kann auch mechanisch und/oder elektrisch in eine Blendschutzvorrichtung selber integriert sein. Falls mechanisch integriert, sind beide miteinander ersetzbar. Falls elektrisch integriert, kann die Energieversorgung und/oder die Lichterfassung und insbesondere die Flackerschaltung gemeinsam genutzt werden. Bei dieser elektrischen Integration kann die Beleuchtungseinrichtung gleichwohl als separate mechanische Baueinheit ausgeführt sein. Die Beleuchtungseinrichtung ist dann über eine feste oder lösbare Signaldrahtverbindung mit der Blendschutzvorrichtung verbunden. Die Blendschutzvorrichtung ist dabei zur Übermittlung eines Messignals entsprechend der erfassten Lichtintensität oder eines Schaltsignals an die Beleuchtungseinrichtung, sobald die Blendschutzvorrichtung einen Lichtbogen detektiert, eingerichtet. Im einfachsten Fall schliesst eine Steuerelektronik der Blendschutzvorrichtung einen Kontakt, über den die Leuchtmittel gespeist sind.

Die Erfassungsmittel und die Leuchtmittel, oder die ganze, als selbständige Einheit ausgeführte Beleuchtungseinrichtung können beispielsweise in einem Stirnbereich oder einem Kinnbereich der Schweisserschutzmaske angeordnet sein. Sie sind jedoch vorzugsweise hinter derselben Vorsatzscheibe wie die Blendschutzvorrichtung angeordnet. Solche austauschbaren Vorsatzscheiben werden üblicherweise zum Schutz von Blendschutzvorrichtungen vor Beschädigung durch Funken und Schlacken eingesetzt. Durch diese Anordnung der Beleuchtungseinrichtung erübrigt sich ein separater Schutz für die Erfassungsmittel und Leuchtmittel, der ebenfalls austauschbar sein müsste. Dadurch wird die Konstruktion der erfindungsgemässen Schweisserschutzmaske vereinfacht.

In einer bevorzugten Ausführungsform der Erfindung weist die Beleuchtungseinrichtung einen Taster auf, mit dem das Einschalten respektive die Inbetriebnahme der Beleuchtungseinrichtung durch Drücken des Tasters realisiert wird. Beim erneuten Drücken des Tasters wird die Beleuchtungseinrichtung wieder ausgeschaltet. Der Taster kann an der Beleuchtungseinrichtung selber oder über eine elektrische Verbindung abgesetzt an beispielsweise einem Schläfenbereich der Schweisserschutzmaske angeordnet sein. Die Beleuchtungseinrichtung wird vorzugsweise automatisch ausgeschaltet, falls während einer vorgegebenen Zeit keine erhöhte Lichtintensität detektiert wird, oder insbesondere die Flackerschaltung nicht anspricht. Dadurch wird ein Entleeren des Energiespeichers vermieden, falls die Beleuchtungseinrichtung irrtümlicherweise nicht abgeschaltet wird.

Eine Beleuchtungseinrichtung gemäss der Erfindung ist zur Verwendung in einer Schweisserschutzmaske gemäss der Erfindung vorgesehen. Diese Beleuchtungseinrichtung weist also die oben im Zusammenhang mit der Schweisserschutzmaske erwähnten Eigenschaften auf. Vorzugsweise ist sie als elektrisch und/oder mechanisch selbständige Baueinheit ausgebildet und in den Adapter einsetzbar..

Ein Adapter gemäss der Erfindung ist zur Aufnahme respektive Halterung einer Beleuchtungseinrichtung gemäss der Erfindung in einer Schweisserschutzmaske ausgestaltet.

Weitere bevorzugte Ausführungsformen gehen aus den abhängigen Patentansprüchen hervor.

### KURZE BESCHREIBUNG DER ZEICHNUNGEN

Im folgenden wird der Erfindungsgegenstand anhand von bevorzugten Ausführungsbeispielen, welche in den beiliegenden Zeichnungen dargestellt sind, näher erläutert. Es zeigen schematisch:
- Figur 1: einen Querschnitt durch eine Schweisserschutzmaske gemäss der Erfindung;
- Figur 2: eine Aufsicht auf einen Adapter gemäss der Erfindung;
- Figur 3: einen Querschnitt durch eine in einen Adapter 2 eingesetzte Beleuchtungseinrichtung; und
- Figur 4: verschiedene Verläufe der Intensität eines abgestrahlten Lichts in Abhängigkeit einer erfassten Umgebungslichtintensität.

Die in den Zeichnungen verwendeten Bezugszeichen und deren Bedeutung sind in der Bezugszeichenliste zusammengefasst aufgelistet. Grundsätzlich sind in den Figuren gleiche Teile mit gleichen Bezugszeichen versehen.

### WEGE ZUR AUSFÜHRUNG DER ERFINDUNG

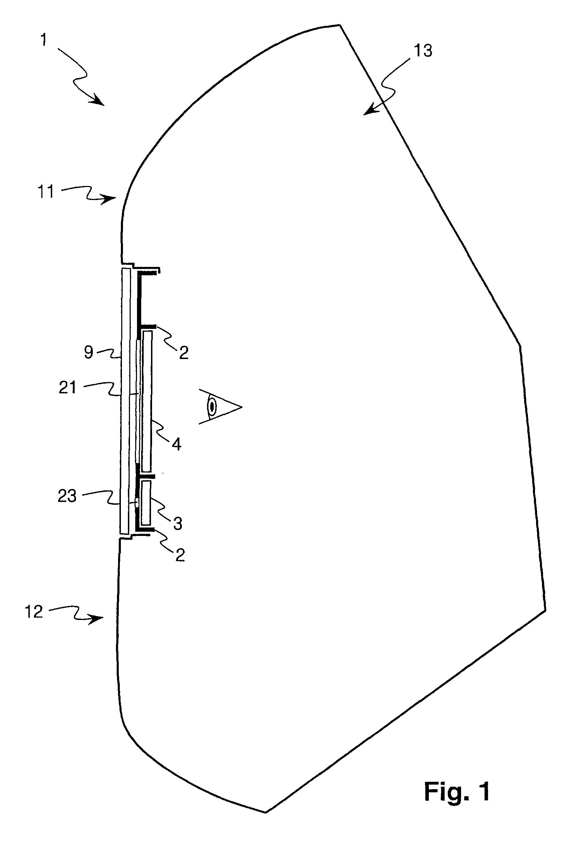

Figur 1 zeigt schematisch einen Querschnitt durch eine Schweisserschutzmaske 1 gemäss der Erfindung. Die Schweisserschutzmaske 1 weist einen Adapter 2 zur Halterung einer Blendschutzvorrichtung 4 und einer erfindungsgemässen Beleuchtungseinrichtung 3 auf. Der Adapter 2 und somit auch die Blendschutzvorrichtung 4 und die Beleuchtungseinrichtung 3 sind hinter einer austauschbaren Vorsatzscheibe 9 angeordnet. Details der Halterung von Adapter 2 und Vorsatzscheibe 9 sowie Blendschutzvorrichtung 4 und Beleuchtungseinrichtung 3 sind nicht eingezeichnet.

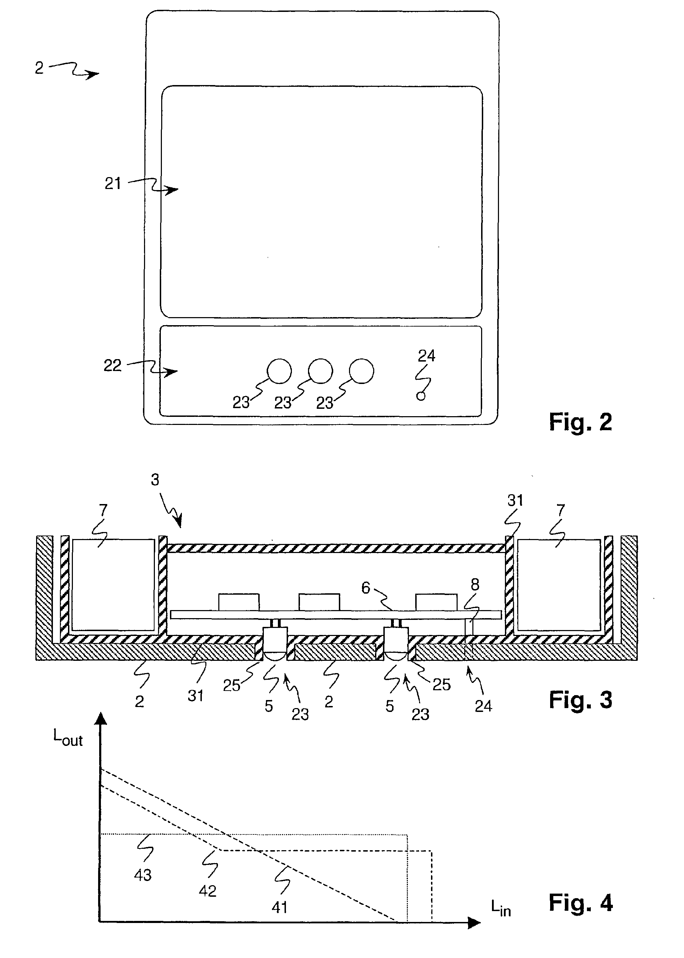

Figur 2 zeigt schematisch eine Aufsicht auf einen Adapter 2 gemäss der Erfindung. Der Adapter 2 weist eine Öffnung 21 zur Aufnahme der Blendschutzvorrichtung 4 und eine Vertiefung 22 zur Aufnahme der Beleuchtungsvorrichtung 3 auf. In der Vertiefung 22 zur Aufnahme der Beleuchtungsvorrichtung 3 sind ein oder mehrere Durchbrüche 23, im vorliegenden Fall drei, für Leuchtmittel, insbesondere Leuchtdioden 5 angeordnet, sowie mindestens ein Durchbruch 24 für Erfassungsmittel 8. Der Adapter 2 weist nicht eingezeichnete Mittel wie z.B. Ein- oder Ausbuchtungen und Schnappelemente oder drehbare oder verschiebbare Verschlusselemente zur lösbaren mechanischen Verbindung des Adapters 2 mit der Maske, der Beleuchtungseinrichtung 3 und der Blendschutzvorrichtung 4 auf.

Figur 3 zeigt schematisch einen Querschnitt durch eine als selbständige Einheit ausgebildete und in einen Adapter 2 eingesetzte Beleuchtungseinrichtung 3. Die Schnittebene verläuft senkrecht zur Bildebene von Figur 2 und durch die mehreren Durchbrüche 23. Es ist eine Ausführungsform mit je zwei Durchbrüchen 23 und Leuchtdioden 5 dargestellt. Die Beleuchtungseinrichtung 3 ist beispielsweise durch eine Schnappverbindung lösbar in den Adapter 2 eingesetzt. Ein korrektes Einsetzen der Beleuchtungseinrichtung 3 in den Adapter 2 liegt vorzugsweise dann vor, wenn ihre nebeneinanderliegenden Ränder bündig sind. Die Beleuchtungseinrichtung 3 weist als Steuermittel 6 eine elektronische Schaltung 6 auf. Als Leuchtmittel 5 sind ein oder mehrere vorzugsweise blaue oder weisse Leuchtdioden 5 eingesetzt. Sie sind vorzugsweise auf eine Platine der elektronischen Schaltung 6 gelötet und ragen durch korrespondierende Durchbrüche in einer Umhüllung 31 der elektronischen Schaltung 6. Um diese Durchbrüche sind Umkragungen 25 in der Umhüllung 31 ausgebildet. Diese Umkragungen 25 korrespondieren mit jeweils zugeordneten Durchbrüchen 23 des Adapters 2, so dass beim Einsetzen der Beleuchtungseinrichtung 3 in den Adapter 2 lichtdichte Abschlüsse entstehen. Die Umkragungen 25 können verspiegelt und als Reflektoren ausgeformt sein. Die Umhüllung 31 ist vorzugsweise ein Gehäuse der Beleuchtungseinrichtung 3 und beispielsweise als Spritzgussteil aus Kunststoff geformt. Die Umhüllung kann auch durch Vergiessen von zumindest der elektronischen Schaltung 6 und der Leuchtdioden 5 mit einem Kunststoff gebildet werden.

Eine als Erfassungsmittel wirkende Photozelle 8, beispielsweise ein Silizium-Photodetektor, liegt in der Figur 3 hinter in der Schnittebene. Die Umhüllung 31 und der Adapter 2 weisen korrespondierende Durchbrechungen auf, so dass die Photozelle 8 Licht vom Arbeitsbereich der Schweisserschutzmaske 1 empfangen kann.

Ein Adapter 2 weist eine oder mehrere Batterien 7 als Energiespeicher auf. Eine Batterie 7 ist entweder einzeln herausnehmbar und ersetzbar, oder wiederaufladbar, oder aber die ganze Beleuchtungseinrichtung 3 weist einen elektrischen Anschluss zum Aufladen der Batterien 7 auf. Optional weist die Beleuchtungseinrichtung 3 auch Solarzellen zur Stützung der Batterieladung auf. Bei einer Ausführung als selbständige Einheit sind entsprechende Durchbrüche für die Solarzellen im Adapter 2 ausgeführt.

Figur 4 zeigt verschiedene bevorzugte Verläufe der Intensität eines abgestrahlten Lichts Lₒᵤₜ in Abhängigkeit einer erfassten Umgebungslichtintensität Lᵢₙ. Gemäss einem ersten Verlauf 41 wird die Intensität des abgestrahlten Lichts von einer maximalen Intensität ausgehend bei zunehmender Intensität des Umgebungslichts monoton auf einen Wert Null verringert.

Gemäss einem zweiten Verlauf 42 wird die Intensität des abgestrahlten Lichts von einer maximalen Intensität ausgehend bei zunehmender Intensität des Umgebungslichts monoton auf einen vorgegebenen Wert verringert, dann konstant gehalten, und beim Erreichen eines weiteren vorgegebenen Werts auf Null gesetzt.

Die kontinuierliche Steuerung respektive Verringerung der abgestrahlten Lichtintensität geschieht durch Modulation einer Speisespannung der Leuchtdioden, und/oder durch eine gepulste Speisung mit einer Pulsbreitenmodulation oder Pulsfrequenzmodulation. Durch die Pulsmodulation ist in einem bestimmten Bereich eine Energieerspamis bei im Wesentlichen gleichbleibender Helligkeit möglich.

Gemäss einem dritten Verlauf 43 wird die Intensität des abgestrahlten Lichts bei zunehmender Intensität des Umgebungslichts konstant gehalten und beim Überschreiten eines bestimmten Schwellwertes oder beim Ansprechen einer Flackerschaltung auf Null gesetzt. Es ist auch möglich, die Lichtintensität gemäss dem ersten oder zweiten Verlauf 41,42 zu regeln, und die Flackerschaltung nur dazu zu verwenden, um beim längeren Ausbleiben eines Schweissvorgangs die Beleuchtungseinrichtung 3 auszuschalten.

Ein Taster oder ein Schalter zum Ein- und Ausschalten der Beleuchtungseinrichtung 3 ist beispielsweise an einem Schläfenbereich 13 der Schweisserschutzmaske 1 angeordnet und über eine Drahtverbindung mit den übrigen Komponenten der Beleuchtungseinrichtung 3 wirkverbunden.

### BEZUGSZEICHENLISTE

- 1: Schweisserschutzmaske
- 2: Adapter
- 3: Beleuchtungseinrichtung
- 4: Blendschutzvorrichtung
- 5: Leuchtmittel, Leuchtdioden
- 6: Steuermittel, Elektronikschaltung
- 7: Batterie
- 8: Erfassungsmittel, Photozelle
- 9: Vorsatzscheibe
- 11: Stirnbereich
- 12: Kinnbereich
- 13: Schläfenbereich
- 21: Öffnung zur Aufnahme der Blendschutzvorrichtung
- 22: Vertiefung zur Aufnahme der Beleuchtungsvorrichtung
- 23: Durchbrüche für Leuchtmittel
- 24: Durchbruch für Erfassungsmittel
- 25: Umkragung
- 31: Umhüllung
- 41: erster Verlauf
- 42: zweiter Verlauf
- 43: dritter Verlauf

## Patentansprüche

1. Schweisserschutzmaske (1) mit einer Beleuchtungseinrichtung (3) zur Ausleuchtung eines Arbeitsbereiches, **dadurch gekennzeichnet, dass** die Beleuchtungseinrichtung (3) aufweist Leuchtmittel (5), Erfassungsmittel (8) zur Erfassung einer Umgebungslichtintensität, einen Energiespeicher (7) zur elektrischen Speisung der Beleuchtungseinrichtung (3) und Steuermittel (6) zur Steuerung einer Intensität eines vom Leuchtmittel (5) abgestrahlten Lichtes nach Massgabe der Umgebungslichtintensität.

2. Schweisserschutzmaske (1) gemäss Anspruch 1, **dadurch gekennzeichnet, dass** die Steuermittel (6) eingerichtet sind, bei zunehmender Umgebungslichtintensität die Intensität des abgestrahlten Lichtes zu verringern.

3. Schweisserschutzmaske (1) gemäss Anspruch 2, **dadurch gekennzeichnet, dass** die Steuermittel (6) eingerichtet sind, mittels einer Flackerschaltung einen Schweissvorgang zu detektieren und das Leuchtmittel (5) während des Schweissvorgangs auszuschalten.

4. Schweisserschutzmaske (1) gemäss einem der Ansprüche 1 bis 3, wo **dadurch gekennzeichnet, dass** bei das Leuchtmittel (5) mindestens eine Leuchtdiode (5) aufweist, und die Steuermittel (6) zur Ansteuerung der mindestens einen Leuchtdiode (5) durch Variation einer Gleichspannung eingerichtet sind.

5. Schweisserschutzmaske (1) gemäss einem der Ansprüche 1 bis 3, **dadurch gekennzeichnet, dass** das Leuchtmittel (5) mindestens eine Leuchtdiode (5) aufweist, und die Steuermittel (6) zur Ansteuerung der mindestens einen Leuchtdiode (5) durch Pulsmodulation eingerichtet ist.

6. Schweisserschutzmaske (1) gemäss einem der vorangehenden Ansprüche, **dadurch gekennzeichnet, dass** die Beleuchtungseinrichtung (3) als herausnehmbare Einheit ausgebildet ist.

7. Schweisserschutzmaske (1) gemäss Anspruch 6, **dadurch gekennzeichnet, dass** die Beleuchtungseinrichtung (3) austauschbar in einen Adapter (2) zur Halterung einer Blendschutzvorrichtung (4) eingesetzt ist.

8. Schweisserschutzmaske (1) gemäss einem der vorangehenden Ansprüche, **dadurch gekennzeichnet, dass** die Beleuchtungseinrichtung (3) einen Taster zur Inbetriebnahme der Beleuchtungseinrichtung (3) aufweist und zur automatischen Ausserbetriebnahme, falls während einer vorgegebenen Zeit kein Schweissvorgang detektiert wird eingerichtet ist.

9. Beleuchtungseinrichtung (3) zur Verwendung in einer Schweisserschutzmaske (1) gemäss einem der Ansprüche 1 bis 8.

10. Adapter zur Halterung einer Beleuchtungseinrichtung (3) gemäss Anspruch 9 in einer Schweisserschutzmaske (1).
